# EUROPEAN PATENT APPLICATION

(11) **EP 4 796 133 A1**
(43) Date of publication of application: **26.08.2026**
(21) Application number: 23956191.3
(22) Date of filing: 20.10.2023
(51) Int. Cl.: A61L 24/00, A61L 24/06

(54) **BONE STRENGTH-CUSTOMIZED BONE CEMENT COMPOSITION**

(30) Priority: 19.10.2023 KR 20230140531
(71) Applicant: ZNEXBIO CO.,LTD., Asan-si, Chungcheongnam-do 31538 (KR)
(72) Inventor: LEE, Byoung Ryol, Cheonan-si, Chungcheongnam-do 31168 (KR)
(74) Representative: Franke, Dirk
(86) International application number: PCT/KR2023/016311
(87) International publication number: WO 2025/084469

(57) **Abstract**

The present invention relates to a bone cement composition comprising polymethylmethacrylate (PMMA) and microbeads, wherein the PMMA and the microbeads satisfy the following conditions: 0.0014x²-0.0221x+0.1816 ≤ P/M ≤ 0.0014x²-0.0165x+2.243 (P is the weight (g) of the polymethylmethacrylate, M is the weight (g) of the microbeads, and x is the bone strength value and in the range of 1.5 to 45). According to the present invention, it is possible to determine a mixing ratio of polymethylmethacrylate and microbeads for providing a bone strength-customized bone cement composition having mechanical properties (strength) similar to the bone strength of a subject to whom bone cement is to be applied. Accordingly, the prepared bone cement composition and bone cement can provide immediate treatment effects when applied to a subject to whom bone cement is to be applied.

## Description

### Technical Field

The present disclosure relates to a bone strength-customized bone cement composition and, more particularly, to a bone cement composition containing polymethyl methacrylate (PMMA) and microbeads, wherein the PMMA and the microbeads are contained in a ratio satisfying a Formula 1: $0.0014 {x}^{2} - 0.0221 x + 0.1816 \leq P / M \leq 0.0014 {x}^{2} - 0.0165 x + 2.243 ,$ where P is a weight (g) of the PMMA, M is a weight (g) of the microbeads, and x is a bone strength value ranging from 1.5 to 45.

### Related Art

Osteoporosis is defined as a skeletal disorder in which decrease in bone strength leads to increase in fracture risk. In recent years, a prevalence of osteoporosis has been rising due to increase in the elderly population, and decline in birth rates, and this growing prevalence of osteoporosis is concerning because it is associated with increase in fracture risk. In the case of femoral fractures, which are the most common types of osteoporotic fractures, it is reported that approximately one out of ten patients dies within one year, and thus such fractures are recognized as a serious public health issue.

Bone cement is a biomaterial adhesive used in orthopedics for reconstruction of a complex fracture and artificial joint surgery, as well as in dentistry for restoring non-vital dentin. Surgical bone cement fills voids between damaged bone and bone, or between bone and an implant, thereby fixing and stabilizing damaged bone and bone, or bone and an implant. Bone cement offers an advantage of a wide range of applications because mixing bone cement components, and applying and curing bone cement are performed within a short period of time, and applying is performed simply. However, when bone cement lacks biocompatibility, it may cause osteocyte necrosis and debonding, making revision surgery unavoidable. Therefore, since bone cement is applied to a patient and ultimately becomes part of an actual bone, the bone cement is fundamentally required to induce bone formation, exhibit excellent stability and adhesion, maintain permanence, and in particular, exhibit mechanical strength similar to that of natural bone.

PMMA-based bone cement has been one of most widely used biomaterial adhesives since it was first introduced in the 1960s as an adhesive between bone and an implant, and is one of most durable materials employed in orthopedic surgery. However, due to intrinsic properties of PMMA, bone union cannot occur, and adverse effects such as interfacial cracking and adjacent site fractures frequently arise, thereby limiting its clinical applicability. Accordingly, many researches have been conducted continuously to overcome drawbacks of PMMA-based bone cement. Conventional technologies to address limitations of existing bone cements include technologies that induce bone regeneration by forming pores and canal structures during biodegradation of biopolysaccharides; technologies that minimize tissue necrosis by reducing effects of heat generated during MMA monomer polymerization, on cells; and technologies that prevent additional fractures by achieving low elastic modulus. Despite development of such diverse technologies, various clinical issues still arise, and thus there remains a need for a bone cement that exhibits excellent bone regeneration capability without causing adverse effects.

Meanwhile, bone strength is determined by approximately 70% bone quantity, and 30% bone quality. Bone quality is determined by factors such as bone turnover rate, structure, presence of microdamage, and mineralization; however, it is currently difficult to measure these factors. In contrast, because bone strength depends on bone quantity, i.e., bone mineral density which accounts for about 70% of bone strength, measurement of the bone mineral density has become known as a method of diagnosing osteoporosis, and a current definition of osteoporosis relies on the bone mineral density.

Bone mineral density can be measured at axial sites of the spine or femur, as well as at peripheral sites, using a common dual-energy X-ray absorptiometry (DXA) method. A T-score of bone mineral density measured at axial sites using DXA serves as a diagnostic criterion for osteoporosis established by the WHO, and this criterion applies only to postmenopausal women, and men aged 50 years or older. However, a substantial proportion of fractures occur outside a T-score range defined for diagnosing osteoporosis, so many fractures are overlooked even when following this criterion.

Another method for measuring bone mineral density involves observing bone resorption parameters such as eroded surfaces and osteoclast counts, in bone tissue morphology. Both viable and non-viable osteoclasts are accumulated in bone tissue, and when non-viable osteoclasts are accumulated in greater numbers than viable osteoclasts, the bone mineral density may still appear high, leading to a diagnosis of low osteoporosis risk. Therefore, despite numerous non-viable osteoclasts are present, elevated bone mineral density value may be derived, thereby overlooking an actual fracture risk. For these reasons, attention has recently been increased to bone strength, beyond bone mineral density, as a cause of osteoporotic fractures. Accordingly, there is a need to provide bone cement that exhibits mechanical strength similar to actual bone strength, based on accurate measurement of bone strength.

Therefore, as a result of extensive research to overcome problems of the prior art, the present inventors have confirmed that, in a bone cement composition containing PMMA and microbeads, when the PMMA and the microbeads are contained in a ratio satisfying Formula 1: $0.0014 {x}^{2} - 0.0221 x + 0.1816 \leq P / M \leq 0.0014 {x}^{2} - 0.0165 x + 2.243 ,$ where P is a weight (g) of the PMMA, M is a weight (g) of the microbeads, and x is a bone strength value ranging from 1.5 to 45, the bone cement composition can be provided exhibiting mechanical properties (strength) similar to a bone strength of a subject to whom the bone cement is to be applied. The present inventors further confirmed that such a composition can provide immediate procedural effectiveness, thereby completing the present disclosure.

### Disclosure

### Technical Problem

Accordingly, a primary object of the present disclosure is to provide a bone strength-customized bone cement composition that exhibits mechanical properties (strength) similar to a bone strength of a subject to whom the bone cement is to be applied, and is capable of providing immediate procedural effectiveness.

Another object of the present disclosure is to provide a method of determining a bone cement composition ratio for defining a mixing ratio of PMMA and microbeads in the above bone strength-customized bone cement composition.

A further object of the present disclosure is to provide a method of manufacturing bone cement, the method including determining a bone cement composition ratio in order to define a mixing ratio of PMMA and microbeads.

### Technical Solution

According to one aspect of the present disclosure, a bone cement composition containing PMMA and microbeads is provided, wherein the PMMA and the microbeads are included in a ratio satisfying Formula 1 below:

$0.0014 {x}^{2} - 0.0221 x + 0.1816 \leq P / M \leq 0.0014 {x}^{2} - 0.0165 x + 2.243 ,$
where
P is a weight (g) of the PMMA,
M is a weight (g) of the microbeads, and
x is a bone strength value, ranging from 1.5 to 45.

Surgical bone cement fills a void between damaged bone and bone, or between bone and an implant, thereby fixing and stabilizing damaged bone and bone, or bone and an implant. Because bone cement applied to a patient functions as an actual part of bone, its inherent strength must be similar to that of natural bone. Accordingly, there is a need to provide bone cement that exhibits mechanical properties similar to a human bone strength, particularly a bone strength of a subject to whom the bone cement is to be applied.

Accordingly, in order to provide bone cement with strength adjusted to varying a bone strength of individual patients, the present inventors investigated factors influencing a strength of a bone cement composition containing PMMA and microbeads. As a result, the present inventors confirmed that a mixing ratio of PMMA to microbeads is a major factor affecting the strength of the bone cement. To prepare bone cement compositions exhibiting various bone strength values ranging 1.5 to 45 MPa, which corresponds to a strength range of human cancellous bone, the present inventors derived Formula 1 for estimating a mixing ratio of PMMA and microbeads, thereby completing the present disclosure.

When the ratio of PMMA to microbeads is below a lower limit of the range defined in Formula 1, it is possible to derive a mixing ratio of PMMA and microbeads capable of manufacturing a bone cement composition exhibiting strength similar to human bone strength; however, moldability of the bone cement decreases, making it difficult to apply to a human body. In contrast, when the ratio exceeds an upper limit of the range defined in Formula 1, the bone cement exhibits strength greater than that of human bone. Therefore, to provide bone cement exhibiting strength similar to human bone strength, it is preferable that PMMA and microbeads be contained in a ratio defined by Formula 1.

According to one experimental example of the present disclosure, the present inventors evaluated whether Formula 1 of the present disclosure can derive an appropriate mixing ratio of PMMA and microbeads for providing a bone cement composition exhibiting a desired bone strength value. As a result, it was confirmed that the bone cement manufactured using the mixing ratio derived from Formula 1 exhibited a bone strength value similar to a desired strength value. In contrast, when the mixing ratio was not derived from Formula 1, or when the ratio fell outside the range defined by Formula 1, it was confirmed that a resulting bone cement did not exhibit a desired bone strength value. These results indicate that the mixing ratio of PMMA and microbeads in the bone cement composition according to the present disclosure, can be adjusted and applied to derive a wide range of bone strength values. This characteristic feature suggests that the present disclosure can provide bone cement compositions exhibiting a desired bone strength value, for example, a bone strength value of a subject to whom the bone cement is to be applied.

In the bone cement composition of the present disclosure, the microbeads may be porous. Because a biodegradation rate of the microbeads must not exceed a growth rate of new bone tissue, it is necessary to regulate the biodegradation rate of the microbeads. Such a biodegradation rate can be regulated through porosity characteristics of the microbeads, and higher porosity, i.e., a higher pore ratio, can increase biodegradability.

In the bone cement composition of the present disclosure, pores of the microbeads may contain hydrogel. The hydrogel serves to enhance an adhesion capability of osteoblasts. The hydrogel may be one or more hydrogels selected from a group consisting of polymer hydrogels such as hyaluronic acid-based hydrogels, collagen-based hydrogels, alginate-based hydrogels, and organic polysaccharide hydrogels, and is preferably a hyaluronic acid-based hydrogel.

In the bone cement composition of the present disclosure, the microbeads may possess biodegradability. For example, the microbeads may be one or more selected from a group consisting of bioglass microspheres; calcium phosphate-based materials such as hydroxyapatite (Ca₁₀(PO₄)₆(OH)₂), α-tricalcium phosphate (α-TCP), β-tricalcium phosphate (β-TCP), biphasic calcium phosphate (BCP, i.e., mixtures of HA and β-TCP with various HA/β-TCP ratios); potassium dihydrogen phosphate; monocalcium phosphate monohydrate (MCPM); dicalcium phosphate (DCP); calcium pyrophosphate (CPC); dicalcium phosphate anhydride (DCPA); dicalcium phosphate dihydrate (DCPD); amorphous calcium phosphate; tetracalcium phosphate (TTCP); Ca(H₂PO₄)₂·H₂O (MCPM); Ca₂P₂O₇; CaHPO₄·2H₂O (DCPD); CaHPO₄ (DCPA); Ca₁₀(PO₄)₆(OH)₂ (HA); tetracalcium phosphate; dicalcium phosphate anhydrous; and dicalcium phosphate dehydrate. Preferably, the microbeads are biodegradable bioglass microspheres. The biodegradability of the microbeads can provide rapid treatment of bone defects by enhancing infiltration of osteoblasts through space, i.e., pores, formed as biodegrade proceeds after implantation.

In the bone cement composition of the present disclosure, a particle size of the biodegradable bioglass microspheres may be 20 to 1000 µm, and preferably 50 to 700 µm. When the particle size of the biodegradable bioglass microspheres is smaller than a lower limit of these ranges, a growth of bone cells becomes difficult, whereas when the particle size of the biodegradable bioglass microspheres exceeds an upper limit of these ranges, it becomes difficult to maintain an appropriate skeletal framework.

In the bone cement composition of the present disclosure, the PMMA may be contained in an amount of 30 to 70 wt% based on a total weight of the composition, and the microbeads may be contained in an amount of 70 to 30 wt% based on a total weight of the composition. When a content of the PMMA is below a lower limit of the range, 30 to 70 wt%, moldability of the bone cement decreases, whereas when the content of PMMA exceeds an upper limit of this range, bone formation becomes difficult. In addition, when a content of the microbeads is below a lower limit of the range, 70 to 30 wt%, bone-forming capability is reduced, whereas the content of the microbeads exceeds an upper limit of this range, moldability of the bone cement decreases.

According to another aspect of the present disclosure, a method of defining a mixing ratio of PMMA and microbeads in the bone cement composition containing PMMA and microbeads is provided, wherein the mixing ratio of PMMA and microbeads is defined according to Formula 1:

$0.0014 {x}^{2} - 0.0221 x + 0.1816 \leq P / M \leq 0.0014 {x}^{2} - 0.0165 x + 2.243 ,$
where
P is a weight (g) of the PMMA,
M is a weight (g) of the microbeads, and
x is a bone strength value, ranging from 1.5 to 45.

In general, a strength of human cancellous bone is known to range from 1.5 to 45MPa, which indicates that bone strength varies from person to person. Because bone cement applied to a human subject ultimately functions as a part of that patient's bone, it is necessary to provide bone cement that exhibits strength matching a bone strength of a subject to whom the bone cement is to be applied. To achieve this, the bone strength of the subject must be predicted, and the mixing ratio of PMMA and microbeads must be defined so as to provide bone cement with strength adjusted to the predicted bone strength.

Prior to defining the mixing ratio of PMMA and microbeads, a preceding step is to predict a bone strength of a subject to whom the bone cement is to be applied. The bone strength of a human subject may be predicted from values inferred through various known measurement methods, and in particular, may be predicted from T-score derived by dual-energy X-ray absorptiometry (DXA), although it is not limited thereto.

As described above, a primary factor influencing the strength of the bone cement is the mixing ratio of PMMA and microbeads. Accordingly, the mixing ratio of PMMA and microbeads may be defined using Formula 1 derived in the present disclosure. Detailed descriptions regarding the mixing ratio of PMMA and microbeads have already been provided above, and will not be repeated here.

According to another aspect of the present disclosure, a method of manufacturing bone cement is provided, the method including: immersing microbeads in hydrogel, drying the microbeads to obtain hydrogel-impregnated microbeads, and mixing the hydrogel-impregnated microbeads with PMMA. The method is characterized in that the mixing ratio of PMMA and the hydrogel-impregnated microbeads is defined according to Formula 1 below:

$0.0014 {x}^{2} - 0.0221 x + 0.1816 \leq P / M \leq 0.0014 {x}^{2} - 0.0165 x + 2.243 ,$
where
P is a weight (g) of the PMMA,
M is a weight (g) of the microbeads, and
x is a bone strength value, ranging from 1.5 to 45.

In the method of manufacturing bone cement of the present disclosure, the hydrogel may be one or more hydrogels selected from a group consisting of hyaluronic acid-based hydrogels, collagen-based hydrogels, alginate-based hydrogels, and organic polysaccharide hydrogels, and is preferably a hyaluronic acid-based hydrogel.

In the method of manufacturing bone cement of the present disclosure, PMMA may be contained in an amount of 30 to 70 wt% based on a total weight of the composition, and the hydrogel-impregnated microbeads may be contained in an amount of 70 to 30 wt% based on a total weight of the composition. When a content of PMMA is below a lower limit of the range, 30 to 70 wt%, moldability of the bone cement decreases, whereas when the content of PMMA exceeds an upper limit of this range, bone formation becomes difficult. In addition, when a content of the microbeads is below a lower limit of the range, 70 to 30 wt%, bone-forming capability is reduced, whereas the content of the microbeads exceeds an upper limit of this range, moldability of the bone cement decreases.

### Advantageous Effects

As described above, the mixing ratio of PMMA and microbeads may be defined so as to provide a customized bone cement composition exhibiting mechanical properties similar to a bone strength of a subject to whom the bone cement is to be applied. Accordingly, the bone cement composition and bone cement prepared in this manner can provide an immediate procedural effectiveness when applied to the subject.

### Best Mode

Hereinafter, the present disclosure will be described in further detail with reference to the following Examples. These Examples are provided only for illustrative purposes, and are not intended to limit the scope of the present disclosure.

### Example: Manufacture of bone cement composition

First, in Formula 1 of the present disclosure, a mixing ratio of PMMA and hydrogel-impregnated microbeads was defined for the case where x is 25, that is, a bone strength value is 25 MPa.$0.0014 {x}^{2} - 0.0221 x + 0.1816 \leq P / M \leq 0.0014 {x}^{2} - 0.0165 x + 2.243$

Specifically, within lower-limit and upper-limit ranges defined by Formula 1, an arbitrary equation (Equation 1) was derived as below, and the mixing ratio (P/M) of the PMMA and the hydrogel-impregnated microbeads was defined by substituting 25 for x in Equation 1: $P / M = 0.0014 {x}^{2} - 0.0221 x + 0.2816$

As a result, P/M was calculated to be approximately 0.6.

Based on this result, a bone cement composition containing PMMA and hydrogel-impregnated microbeads at a P/M ratio of 0.6 was manufactured. Briefly, hyaluronic acid was dissolved in deionized water to prepare a hydrogel at a concentration of 10 mg/mL, and 0.5 g of TCP microbeads was immersed therein to allow the hydrogel to infiltrate pores of the microbeads, thereby preparing hydrogel-impregnated microbeads. PMMA and the hydrogel-impregnated microbeads were then weighed to achieve a P/M ratio of 0.6 (40wt% PMMA and 60wt% hydrogel-impregnated microbeads), followed by mixing to manufacture the bone cement composition.

### Comparative Example 1: Manufacture of bone cement composition

A bone cement composition having a P/M ratio of 9, rather than the P/M ratio of 0.6 derived from Equation 1, was prepared. Briefly, hyaluronic acid was dissolved in deionized water to prepare a hydrogel at a concentration of 10 mg/mL, and 0.5 g of TCP microbeads was immersed therein to allow the hydrogel to infiltrate pores of the microbeads, thereby preparing hydrogel-impregnated microbeads. PMMA and the hydrogel-impregnated microbeads were then weighed to achieve a P/M ratio of 9 (90wt% PMMA and 10wt% hydrogel-impregnated microbeads), followed by mixing to manufacture the bone cement composition.

### Comparative Example 2: Manufacture of bone cement composition

When a bone strength value is 25MPa, a mixing ratio of PMMA and microbeads was defined by substituting 25 for x in 0.0014x²-0.0165x+2.243, an upper limit equation of Formula 1, and P/M was calculated to be approximately 2.7. Based on this result, a bone cement composition having a P/M ratio of 2.7 was manufactured. Briefly, hyaluronic acid was dissolved in deionized water to prepare a hydrogel at a concentration of 10 mg/mL, and 0.5g of TCP microbeads was immersed therein to allow the hydrogel to infiltrate pores of the microbeads, thereby preparing hydrogel-impregnated microbeads. PMMA and the hydrogel-impregnated microbeads were then weighed to define a P/M ratio of 2.7 (73wt% PMMA and 27wt% hydrogel-impregnated microbeads), followed by mixing to manufacture the bone cement composition.

### Experimental Example: Measurement of bone strength

Using the bone cements manufactured in the above Example and Comparative Examples 1 and 2, bone-shaped specimens were arbitrarily formed, and their strength was measured. The results are shown in Table 1.

**[Table 1]**

| | **Strength (Mpa)** |
|---|---|
| **Example** | 23.7 |
| **Comparative Example 1** | 77.4 |
| **Comparative Example 2** | 49.7 |

As shown in Table 1, it was confirmed that the bone cement manufactured by applying the mixing ratio of PMMA and hydrogel-impregnated microbeads defined by Formula 1 of the present invention, exhibited strength similar to the desired bone strength of 25 MPa. In contrast, it was confirmed that the bone cement manufactured by applying a mixing ratio not derived from the Formula 1 of the present invention (Comparative Example 1), exhibited strength at least three times higher than a desired bone strength of 25MPa. Furthermore, when a mixing ratio was outside the range of Formula 1 of the present invention (Comparative Example 2), a mixing ratio different from that of the Example was obtained, even at the same bone strength. In addition, the bone cement prepared by applying such a mixing ratio, exhibited strength different from a desired bone strength of 25MPa.

## Claims

1. A bone cement composition containing polymethyl methacrylate (PMMA) and microbeads, wherein the PMMA and the microbeads are contained in a ratio satisfying Formula 1 below: $0.0014 {x}^{2} - 0.0221 x + 0.1816 \leq P / M \leq 0.0014 {x}^{2} - 0.0165 x + 2.243$ where
P is a weight (g) of the PMMA,
M is a weight (g) of the microbeads, and
x is a bone strength value, ranging from 1.5 to 45.

2. The bone cement composition of claim 1, wherein the microbeads are porous.

3. The bone cement composition of claim 2, wherein pores of the microbeads contain hydrogel.

4. The bone cement composition of claim 3, wherein the hydrogel is one or more hydrogels selected from a group consisting of polymer hydrogels such as hyaluronic acid-based hydrogels, collagen-based hydrogels, alginate-based hydrogels, and organic polysaccharide hydrogels.

5. The bone cement composition of claim 1, wherein the microbeads are selected from a group consisting of bioglass microspheres; calcium phosphate-based materials such as hydroxyapatite (Ca₁₀(PO₄)₆(OH)₂), α-tricalcium phosphate (α-TCP), β-tricalcium phosphate (β-TCP), biphasic calcium phosphate (BCP, i.e., mixtures of HA and β-TCP with various HA/β-TCP ratios); potassium dihydrogen phosphate; monocalcium phosphate monohydrate (MCPM); dicalcium phosphate (DCP); calcium pyrophosphate (CPC); dicalcium phosphate anhydride (DCPA); dicalcium phosphate dihydrate (DCPD) ; amorphous calcium phosphate; tetracalcium phosphate (TTCP) ; Ca(H₂PO₄)₂·H₂O (MCPM); Ca₂P₂O₇; CaHPO₄·2H₂O (DCPD) ; CaHPO₄ (DCPA) ; Ca₁₀(PO₄)₆(OH)₂ (HA) ; tetracalcium phosphate; dicalcium phosphate anhydrous; and dicalcium phosphate dehydrate.

6. The bone cement composition of claim 5, wherein a particle size of the biodegradable bioglass microspheres is 20 to 1000µm.

7. The bone cement composition of claim 1, wherein the PMMA is contained in an amount of 30 to 70 wt% based on a total weight of the composition, and the microbeads is contained in an amount of 70 to 30 wt% based on a total weight of the composition.

8. A method of defining a mixing ratio of polymethyl methacrylate (PMMA) and microbeads in a bone cement composition containing the PMMA and microbeads of claim 1, wherein the mixing ratio of PMMA and microbeads is defined according to Formula 1: $0.0014 {x}^{2} - 0.0221 x + 0.1816 \leq P / M \leq 0.0014 {x}^{2} - 0.0165 x + 2.243 ,$ where
P is a weight (g) of the PMMA,
M is a weight (g) of the microbeads, and
x is a bone strength value, ranging from 1.5 to 45.

9. A method of manufacturing bone cement, the method comprising: immersing microbeads in hydrogel, drying the microbeads to obtain hydrogel-impregnated microbeads, and mixing the hydrogel-impregnated microbeads with polymethyl methacrylate (PMMA), wherein a mixing ratio of the PMMA and hydrogel-impregnated microbeads is defined by the method of claim 8.

10. The method of manufacturing bone cement of claim 9, wherein the hydrogel is one or more hydrogels selected from a group consisting of polymer hydrogels such as hyaluronic acid-based hydrogels, collagen-based hydrogels, alginate-based hydrogels, and organic polysaccharide hydrogels.

11. The method of manufacturing bone cement of claim 9, wherein the PMMA is contained in an amount of 30 to 70 wt% based on a total weight of the composition, and the microbeads is contained in an amount of 70 to 30 wt% based on a total weight of the composition.
